Europäisches Patentamt

European Patent Office

Office européen des brevets

(11) Veröffentlichungsnummer: **0 321 732**
**A1**

(12) # EUROPÄISCHE PATENTANMELDUNG

(21) Anmeldenummer: **88119737.0**

(22) Anmeldetag: **26.11.88**

(51) Int. Cl.⁴: **A41B 13/02**

(30) Priorität: **10.12.87 DE 3741828**

(43) Veröffentlichungstag der Anmeldung:
**28.06.89 Patentblatt 89/26**

(84) Benannte Vertragsstaaten:
**AT BE CH DE ES FR GB IT LI NL SE**

(71) Anmelder: **Paul Hartmann Aktiengesellschaft**
**Paul-Hartmann-Strasse**
**D-7920 Heidenheim(DE)**

(72) Erfinder: **Malowaniec,Krzysztof, Dipl.-Ing.**
**Nürtinger Weg 10**
**D-7920 Heidenheim(DE)**

(74) Vertreter: **Becker, Maria, Dipl.-Phys.**
**Auf dem Haigst 29**
**D-7000 Stuttgart 70(DE)**

(54) **Wegwerfwindel sowie Verfahren und Vorrichtung zur Herstellung einer Wegwerfwindel.**

(57) Bei einer Wegwerfwindel, wie Höschenwindel, mit sich seitlich der Ränder des Saugkissens (28) zwischen Wäscheschutzfolie (10) und Vliesstoffabdeckung (36) erstreckenden mehreren elastischen Bändern (34) (Bandgruppe) wird vorgeschlagen, dass jeweils mindestens einer der Bänder gegenüber den übrigen Bändern eine unterschiedliche Vorspannung aufweist. Wenn das dem Saugkörper benachbarte Band (34a, 34b) gegenüber den zur gleichen Gruppe gehörenden Bändern (34c, 34d) die stärkste Vorspannung aufweist, wird das Saugkissen gegenüber den Seitenlaschen leicht umgestülpt und ergibt die gewünschte Säckchenbildung, während die elastischen Bänder mit der geringeren Vorspannung am Schenkel abdichtend und ohne starke Druckausübung anliegen.

Die Erfindung sieht ferner ein Verfahren zur Herstellung einer solchen Wegwerfwindel und eine Vorrichtung zur Durchführung des Verfahrens vor.

Fig. 1

EP 0 321 732 A1

## Wegwerfwindel sowie Verfahren und Vorrichtung zur Herstellung einer Wegwerfwindel

Die Erfindung betrifft eine als Höschen anlegbare Wegwerfwindel nach dem Oberbegriff des Anspruchs 1 sowie ein Verfahren und eine Vorrichtung zur Herstellung dieser Wegwerfwindel.

Aus dem deutschen Gebrauchsmuster G 83 06 466.4 ist eine Wegwerfwindel mit im Beinausschnitt angeordneten elestischen Teilen bekannt, bei welcher die elastischen Teile jeweils aus mindestens drei im wesentlichen nebeneinander angeordneten elastisch-dehnbaren Fäden besteht. Die Fäden haben sämtlich eine gleiche Dehnungsstärke. Dadurch die Vielzahl der Fäden eine breitere elastische Strecke entsteht, bewirkt dies, dass am Schenkel eine breite, elastische Fläche auf der Haut des Kindes aufliegt und das Bein umschliesst.

Aus der EP-A-0121178 ist ein Verfahren zur Herstellung einer Höschenwindel mit mehreren elastischen Bändern bekannt geworden, die mit einer unterschiedlichen Spannung in ihrer Längsrichtung ausgestattet werden. Dabei wird die Spannung der einzelnen elastischen Fäden periodisch geändert derart, dass die Spannung der Fäden im Schrittbereich einen Maximalwert und im Taillenbereich einen Minimalwert aufweist. Die Spannung der einzelnen parallel nebeneinanderliegenden Fäden ist die gleiche. Es ist nicht vorgesehen, die Spannung der Bänder gegenüber den benachbarten Bändern zu verändern.

Das Gleiche gilt bei der Höschenwindel nach der EP-A-0115286. Hier ist wie beim eingangs erwähnten Gebrauchsmuster G 83 06 466.4 an jeder Seite des Saugkörpers eine Schar von elastischen Bändern vorgesehen, die jeweils gleiche Dehnungsstärke haben. Sie werden aus einem breiteren elastischen Band mittels einer Trennvorrichtung in einzelne Gummifäden aufgeteilt. Durch die Verwendung mehrerer paralleler Gummifäden mit gleicher Dehnstärke sollen lineare Druckstellen am Oberschenkel des Kindes vermieden werden.

Das Aufbringen von elastischen Bändern innerhalb der Längsrandbereiche von Wegwerfwindeln ist auch in der EP-A-0027303 beschrieben. Um im Schrittbereich eine Raffung der Windel zu erreichen, werden die elastischen Fäden bei der Herstellung der Windel jeweils abwechselnd gestreckt und entspannt, wobei die entspannten Teile der Gummifäden im Taillenbereich und die gestreckten Teile im Schrittbereich liegen. Auch bei dieser Druckschrift ist lediglich eine periodische Änderung der Vorspannung der jeweiligen Bänder in Längsrichtung geoffenbart.

Der Erfindung liegt die Aufgabe zugrunde eine als Höschen anlegbare Wegwerfwindel zu schaffen, die ein angenehmes Tragen mit körpergerechtem Abschluss der Höschenwindel im Schenkel- und/oder Taillenbereich ohne Ausübung von Druckstellen ermöglicht und die ein besonders gutes Abdichten gewährleistet, wobei die Windel gleichzeitig eine gewünschte Säckchenbildung im Schrittbereich aufweist. Ausserdem soll die Höschenwindel serienmässig fertigungstechnisch einfach herzustellen sein.

Diese Aufgabe wird gemäss der Erfindung dadurch gelöst, dass jeweils mindestens eines der zu einer Gruppe gehörenden elastischen Bänder gegenüber den übringen Bändern eine andere Vorspannung aufweist, wobei das dem Saugkörper benachbarte elastische Band gegenüber den zur gleichen Gruppe gehörenden Bändern die grösste Vorspannung aufweist. Unter Gruppe sind diejenigen Bänder zu verstehen, die sich jeweils zwischen und in Längsrichtung von Randbereichen vom Saugkörper und zwischen den Windelabdeckungen (Wäscheschutzfolie und Vliesstoff) erstrecken.

Hierdurch kann erreicht werden, dass sich die Windelhose in gewünschter Weise in Gebrauchslage so zusammenzieht, dass eine Säckchenbildung entsteht. Wenn der Abstand zwischen dem Saugkörper und dem diesem benachbarten elastischen Band zumindest der Dicke des Saugkörpers in dessen Randbereich entspricht, wird sich das stärker gespannte elastische Band am Saugkörper beim Gebrauch anlegen, so dass dieses beim Tragen nicht spürbar ist. Die weniger gespannten elastischen Bänder befinden sich aussen und bilden einen angenehmen Beinverschluss, ohne Druckstellen auszuüben.

Ein vorteilhaftes Verfahren zur Herstellung einer Wegwerfwindel nach der Erfindung ist Gegenstand des Anspruchs 6.

Eine einfache Vorrichtung zur Durchführung des Verfahrens ist Gegenstand der Ansprüche 7 bis 9.

In der Zeichnung ist ein Ausführungsbeispiel der Erfindung dargestellt. Es zeigen:

Fig. 1 eine teilweise aufgebrochene Darstellung einer flachgelegten Wegwerfwindel;

Fig. 2 einen Teilschnitt entlang der Linie 2-2 der Fig. 1;

Fig. 3 Einzelheit gemäss Fig. 2, in Gebrauchszustand;

Fig. 4 in schematischer Darstellung eine Vorrichtung zur Aufbringung der verschiedene Vorspannung aufweisenden elastischen Bänder auf die Wegwerfwindel.

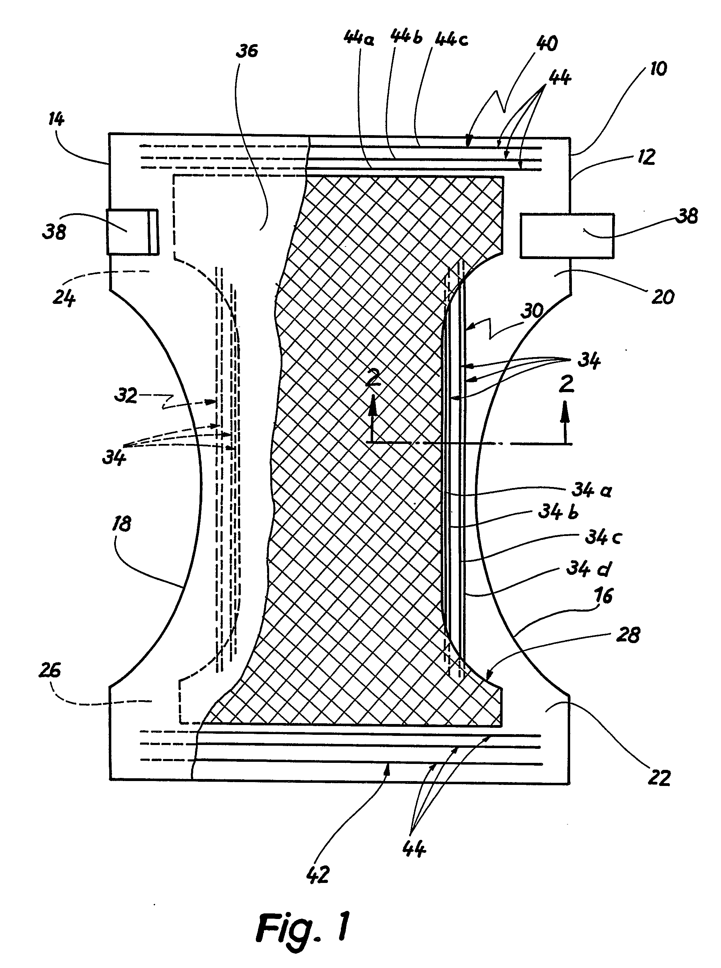

Die dargestellte Wegwerfwindel ist an ihrer einen, in Fig. 1 unteren Seite mit einer Wäscheschutzfolie 10 ausgestattet, die vorzugsweise aus Polyäthylen besteht. Sie bildet beispielsweise einen im wesentlichen rechteckförmigen Zuschnitt, der im mittleren Bereich seiner beiden Längskanten 12, 14 jeweils einen Beinausschnitt 16 bzw. 18 aufweist. Die beiden seitlich dieser Beinausschnitte 16, 18 liegenden Folienabschnitte 20, 22 bzw. 24, 26 umschliessen in angelegtem Zustand der Höschenwindel den Körper im Bereich seiner Taille.

Mit 28 ist als Ganzes ein Saugkörper bezeichnet, der den Umrissen des Folienzuschnittes in kleineren Abmessungen in etwa entspricht. Dieser besteht beispielsweise aus einem Zellulosefasergemisch. Im Schrittbereich kann der Saugkörper 28 noch mit einer dicker auftragenden, vermehrt flüssigkeitsabsorbierenden Verstärkung versehen sein, die im wesentlichen rechteckförmig ist und im Schrittbereich vorzugsweise von den Saugkörperrandteilen mit geringem Abstand endet und mit dem Saugkörpermaterial homogen ist.

Wie aus Fig. 1 ersichtlich ist, ist jedem Längsrandteil im Bereich der Beinausschnitte 16 und 18 eine als Ganzes mit 30 bzw. 32 bezeichnete Gruppe von fadenförmigen, elastischen Bändern 34 zugeordnet, die sich parallel zur Längsrichtung der Wegwerfwindel erstrecken, in seitlichem Abstand voneinander angeordnet und auf die Innenseite der Wäscheschutzfolie 10, beispielsweise durch Kleben, aufgeheftet sind. 36 bezeichnet eine sowohl die Wäscheschutzfolie 10 innenseitig als auch den Saugkörper 29 überdeckende Vliesstoffabdeckung 36, welche auch die Bänder 34 zwischen sich und der Wäscheschutzfolie 10 einschliesst.

An den einander gegenüberliegenden Folienabschnitten 20 und 24 der Wäscheschutzfolie 10 sind noch an sich bekannte Verschlusslaschen 38 befestigt, mit denen sich in angelegtem Zustand der Wegwerfwindel diese Folienabschnitte mit den entsprechend zugeordneten Folienabschnitten 22 bzw. 26 miteinander verbinden lassen.

Wie Fig. 1 zeigt, ist ausserdem jeder Querseite des Saugkörpers 28 eine weitere Gruppe 40 bzw. 42 von fadenförmigen, elastischen Bändern 44 zugeordnet, die gleichfalls auf die Innenseite der Wäscheschutzfolie 10 aufgeheftet sind.

Die Bänder 34 bzw. 44 der Bandgruppen 30, 32, 40, 42 dienen dazu, die Wegwerfwindel in am Körper angelegtem Zustand im Schenkel- und Taillenbereich elastisch nachgiebig anzulegen.

In diesem Zusammenhang sei bemerkt, dass zumindest die Anordnung der Bänder 44 im Taillenbereich nicht zwingend und es ggf. auch denkbar ist, nur eine der beiden Bandgruppen 40 bzw. 42 vorzusehen.

Von den Bändern 34 bzw. 44 der einzelnen Bandgruppen 30, 32, 40, 42 ist jeweils mindestens ein Band stärker vorgespannt als die übrigen zur gleichen Gruppe gehörigen Bänder. Im vorliegenden Falle weisen die beiden Bandgruppen 30, 32 jeweils vorzugsweise vier einzelne elastische Bänder 34 auf, von denen die beiden, dem Saugkörper 28 am nächsten liegenden, Bänder 34a und 34b eine grössere Vorspannung aufweisen als die beiden restlichen Bänder 34c und 34d. Ebenso weist das dem Saugkörper nächstliegende elastische Band 44a eine grössere Vorspannung auf als die beiden anderen Bänder 44b und 44c.

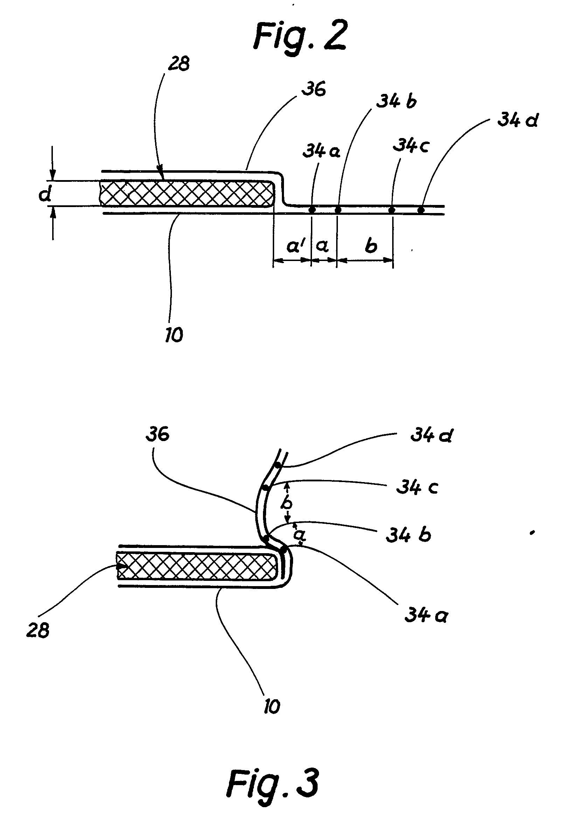

Wie Fig. 2 zeigt, ist in bevorzugter Weise der Abstand zwischen dem Saugkörper 28 und dem diesem benachbarten, elastischen Band 34a der beiden Bandgruppen 30, 32 zumindest entsprechend der Dicke d des Saugkörpers 28 in dessen randbereich ausgelegt. Wie Fig. 2 zeigt, ist der Abstand a' sogar etwas grösser gewählt, während der Abstand a des Bandes 34a zum benachbarten Band 34b lediglich einem Bruchteil des Abstandes a' entspricht. Diese beiden Bänder 34a und 34b weisen vorzugsweise die gleiche Vorspannung auf, die grösser ist als diejenige der Bänder 34c und 34d. Zwischen diesem Bandpaar und dem Band 34b ist ein Abstand b gewählt, der vorzugsweise grösser ist als der Abstand a. Durch die Wahl dieser Abstände a', a, b und die unterschiedlich grosse Vorspannung der beiden Bandpaare wird, wie aus Fig. 3 ersichtlich ist, der Effekt bewirkt, dass sich aufgrund der Vorspannung der innenliegenden Bänder 34a und 34b das die beiden Bandpaare aufnehmende Randstück der Wäscheschutzfolie 10 mit der Vliesstoffabdeckung 36 und dem Längskantenbereich des Saugkörpers 28 nach innen umlegt. Dies bewirkt eine gewünschte Säckchenbildung einerseits und ein angenehmes Anschmiegen der Seitenklappen der Wegwerfwindel.

Fig. 3 verdeutlicht ausserdem, dass aufgrund der erhöhten Vorspannung der Bänder 34a und 34b diese auf dem Saugkörper 28 aufruhen und somit nicht in unangenehmer Weise am Körper bzw. Schenkel spannen. Die erfindungsgemässe Konstruktion bewirkt somit,dass die beiden aussenliegenden Bänder 34c und 34d relativ sanft am Körper anliegen und keinen unangenehmen Druck ausüben.

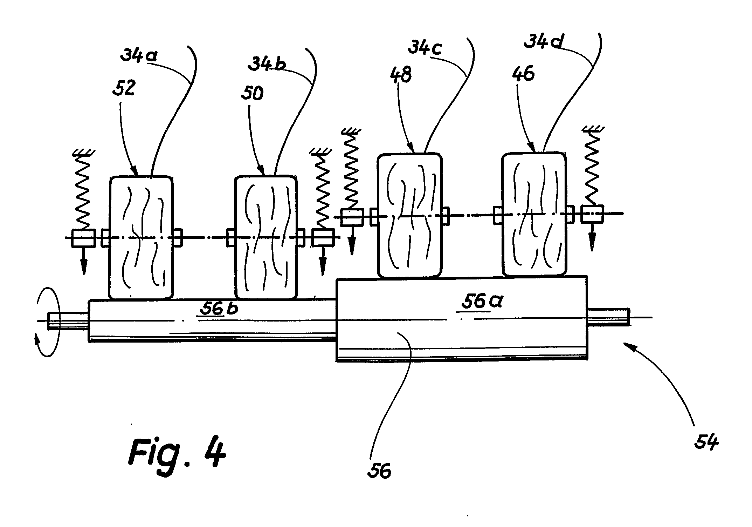

Die Fig. 4 zeigt einen Teil einer Vorrichtung, mit deren Hilfe sich jeweils eine Gruppe 30 bzw. 32 der elastischen Bänder 34 verfahrenstechnisch einfach mit unterschiedlicher Vorspannung auf die Wäscheschutzfolie 10 aufbringen lassen.

Hierzu werden zunächst die einzelnen Bänder 34 zu Bandspulen 46, 48, 50 und 52 aufgespult und dann durch die Vorrichtung paarweise mit unterschiedlicher Umfangsgeschwindigkeit wieder abgespult. Im vorliegenden Falle wird dies in einfacher Weise dadurch erreicht, dass alle Bandspulen 46, 48 50 und 52 auf einen gleichen Durchmesser gewickelt, jedoch mit unterschiedlicher Umfangsgeschwindigkeit wieder abgespult werden. Dies wird in technisch einfacher Weise dadurch bewerkstelligt, dass eine Abspulvorrichtung 54 vorgesehen ist, die eine Antriebswelle 56 mit Abschnitten 56a und 56b unterschiedlichen Durchmessers aufweist. Die Spulen 46, 48 der mit grösserer Vorspannung aufzubringenden Bänder 34a und 34b werden hierzu ständig mit dem Umfang des einen kleineren Durchmesser aufweisenden Wellenabschnittes 56b in Berührung gehalten, während die mit schwächerer Vorspannung aufzubringenden Bänder 34c und 34d an den einen grösseren Durchmesser aufweisenden Wellenabschnitt 56a unter Druck angelegt werden, so dass bei Rotation der Antriebswelle 56 durch Friktion die einzelnen Spulen mitgenommen werden.

Es ist klar, dass auch die Gruppen 40 und 42 der Bänder 44 auf die gleiche Art und Weise aufgebracht werden können. Selbstverständlich ist es auch denkbar, dass jede einzelne Spule mit einer unterschiedlichen Umfangsgeschwindigkeit abgespult und dementsprechend von Band zu Band die Vorspannung unterschiedlich ist.

**Ansprüche**

1. Als Höschen anlegbare Wegwerfwindel, mit einer in angelegtem Zustand aussen liegenden, flüssigkeitsundurchlässigen Wäscheschutzfolie, einer innenseitig aufgebrachten,die Wäscheschutzfolie abdeckenden Vliesstoffabdeckung, einem zwischen beiden angeordneten Saugkörper, der sich in Längs- und Querrichtung im Abstand von den entsprechenden Randteilen von Wäscheschutzfolie und Vliesstoffabdeckung befindet und mit mehreren, nebeneinander liegenden, zu jeweils einer Gruppe zusammengefassten, sich jeweils zwischen und in Längsrichtung von Randbereichen von Saugkörper und Vliesstoffabdeckung erstreckenden,mit mindestens einem der sie abdeckenden Teile verbundenen, fadenförmigen, elastischen Bändern,
**dadurch gekennzeichnet,** dass jeweils mindestens eines der zu einer Gruppe (30, 32, 40, 42) gehörenden elastischen Bänder (34, 44) gegenüber den übrigen Bändern eine andere Vorspannung aufweist, wobei das dem Saugkörper (28) benachbarte elastische Band (34a, 44a) gegenüber den zur gleichen Gruppe gehörenden Bändern (34 bzw. 44) die grösste Vorspannung aufweist.

2. Wegwerfwindel nach Anspruch 1, dadurch gekennzeichnet, dass der Abstand a zwischen dem Saugkörper (28) und dem diesem benachbarten elastischen Band (34a; 44a) der einzelnen Bandgruppen (34; 44) zumindest der Dicke d des Saugkörpers (28) in dessen Randbereich entspricht.

3. Wegwerfwindel nach Anspruch 1 oder 2, dadurch gekennzeichnet, dass dem Saugkörper (28) an jeweils einer Längsseite eine Gruppe (30 bzw. 32) von mindestens vier elastischen Bändern (34a, 34b, 34c, 34d) zugeordnet ist, wobei von jeder Gruppe (30; 32) stärker vorgespannte Bänder (34a und 34b) nahe am Saugkörper (28) liegen und dass der Abstand a der beiden saugkörpernahen Bänder (34a und 34b) jeder Bandgruppe voneinander kleiner ist als der Abstand b zwischen beiden Bandpaaren (34a, 34b und 34c, 34d).

4. Wegwerfwindel nach Anspruch 1, dadurch gekennzeichnet, dass dem Saugkörper (28) an jeweils einer Querseite (Taillenbereich) eine Gruppe (40; 42) von mindestens zwei elastischen Bändern (44a und 44b) unterschiedlicher Vorspannung zugeordnet ist.

5. Wegwerfwindel nach einem der vorhergehenden Absprüche, dadurch gekennzeichnet, dass jedes der zu einer Gruppe (30, 32; 40, 42) gehörenden elastischen Bänder (34; 44) aus mindestens zwei parallelen elastischen Fäden besteht.

6. Verfahren zur Herstellung einer Wegwerfwindel nach einem der vorhergehenden Ansprüche, dadurch gekennzeichnet, dass die zu jeweils einer Bandgruppe (30; 32; 40; 42) gehörenden Bänder (34; 44) einzeln zu einer Spule (46; 48; 50; 52) gewickelt werden, die mit unterschiedlichen Umfangsgeschwindigkeiten abgespult und auf die Wegerfwindel aufgebracht werden.

7. Vorrichtung zur Durchführung des Verfahrens nach Anspruch 6, gekennzeichnet durch eine die Spulen (46; 48; 50; 52) jeder Bandgruppe (30; 32) mit unterschiedlicher Umfangsgeschwindigkeit antreibenden Abspulvorrichtung (54).

8. Vorrichtung nach Anspruch 7, dadurch gekennzeichnet, dass die Abspulvorrichtung (54) eine Antriebswelle (56) mit Abschnitten (56a und 56b) unterschiedlichen Durchmessers aufweist, an welche die Spulen (46; 48; 50; 52) mit ihrem Umfang ständig anliegen.

9. Vorrichtung nach Anspruch 8, dadurch gekennzeichnet, dass die Antriebswelle (56) zwei Abschnitte (56a; 56b) unterschiedlichen Durchmessers aufweist und dass an jedem Wellenabschnitt (56a; 56b) ein Spulenpaar (46; 48 bzw. 50; 52) anliegt.

**Fig. 1**

Fig. 2

Fig. 3

Fig. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4 ) |
|---|---|---|---|
| X | US-A-4300562 (PIENIAK) <br> * Spalte 2, Zeile 6 - Zeile 58; Ansprüche 5-22; Figuren 1-12 * | 1 | A41B13/02 |
| A | | 2-5 | |
| | --- | | |
| X | US-A-4371417 (FRICK ET AL) <br> * Spalte 3, Zeile 30 - Zeile 35; Figur 4 * | 6, 7 | |
| | --- | | |
| A | DE-A-3423644 (PAUL HARTMANN) <br> * Anspruch 1; Figur 1 * | 1-5 | |
| | --- | | |
| A | EP-A-235815 (KIMBERLY-CLARK CORPORATION) <br> * Zusammenfassung; Figuren 1-6 * | 1-7 | |
| | --- | | |
| D,A | EP-A-115286 (BOUSSAC SAINT FRERES B.S.F. S.A.) <br> * Zusammenfassung; Figuren 1-8 * | 1-7 | |
| | ----- | | |

**RECHERCHIERTE SACHGEBIETE (Int. Cl.4 )**

A41B
A61F

Der vorliegende Recherchenbericht wurde für alle Patentansprüche erstellt

| Recherchenort <br> DEN HAAG | Abschlußdatum der Recherche <br> 13 APRIL 1989 | Prüfer <br> KARIPIDOU C. |
|---|---|---|

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
............................................................
& : Mitglied der gleichen Patentfamilie, übereinstimmendes Dokument

EPO FORM 1503 03.82 (P0403)